# EUROPEAN PATENT SPECIFICATION

(11) **EP 1 318 142 B1**
(45) Date of publication and mention of the grant of the patent: **11.04.2007**
(21) Application number: 01963437.7
(22) Date of filing: 05.09.2001
(51) Int. Cl.: C07C 271/20, C07C 269/06, C07K 14/00, B01J 39/04, B01J 39/26

(54) **T-BUTOXYCARBONYLAMINOETHYLAMINE FOR THE SYNTHESIS OF PNA MONOMER UNITS, AMINO ACID DERIVATIVES, INTERMEDIATES THEREOF, AND PROCESSES FOR PRODUCTIONS OF THEM**
T-BUTOXYCARBONYLAMINOETHYLAMINE FÜR DIE HERSTELLUNG VON PNA-MONOMEREINHEITEN, AMINOSÄUREDERIVATE, ZWISCHENPRODUKTE UND VERFAHREN ZU DEREN HERSTELLUNG
T-BUTOXYCARBONYLAMINOETHYLAMINE DESTINE A LA SYNTHESE D'UNITES MONOMERE PNA, DERIVES D'AMINO ACIDE, INTERMEDIAIRE DE CEUX-CI ET PROCESSUS DE PRODUCTION DE CES DERIVES

(30) Priority: 05.09.2000 JP 2000268638
(43) Date of publication of application: 11.06.2003
(73) Proprietor: Credia Japan Co., Ltd., Soraku-gun, Kyoto 619-0273 (JP)
(72) Inventor: IKEDA, Hisafumi, Nagareyama-shi, Chiba 270-0115 (JP); SAITO, Isao, Kyoto-shi, Kyoto 607-8242 (JP); NAKAMURA, Yushin, Kashiwa-shi, Chiba 277-0041 (JP)
(74) Representative: Perry, Robert Edward
(86) International application number: PCT/JP2001/007696
(87) International publication number: WO 2002/020470

(56) References cited:
- WO-A-98/37232
- WO-A1-97/30053
- WO-A2-99/31121
- US-A- 5 595 741
- ROESKE ROGER W. ET AL.: 'Selective reduction of the amide carbonyl group in dipeptides by borane' J. ORG. CHEM. vol. 41, no. 7, 1976, pages 1260 - 1261, XP002906783

## Description

### Technical Field of the Invention

The invention relates to a process for amino acid derivatives and t-butoxycarbonylaminoethylamine which is their intermediate, and more particularly relates to a process for amino acid derivatives and t-butoxycarbonylaminoethylamine which is their intermediate, expediently used as a base or a base substance for introducing a functional molecule in case of synthesizing a monomer unit for syntheses of Boc-type PNA, an amino acid derivative introducing a Boc-type functional molecule and the like.

### Background Art

Amino acid derivatives shown by the below formula (I) 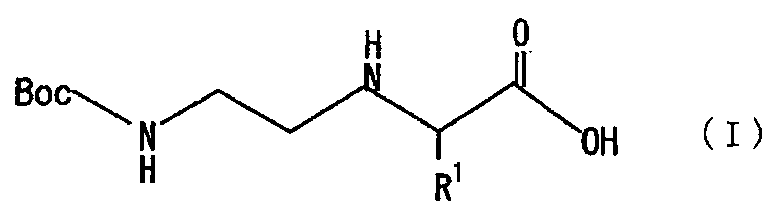 (wherein R¹ means a hydrogen atom or a straight chain or branched chain alkyl groups with 1-5 carbon atoms. Hereinafter it is same.) have a wide variety of use as a base or a base substance for introducing a functional molecule in case of synthesizing a monomer unit for synthesis of Boc-type PNA, an amino acid derivative introducing a Boc-type functional molecule and the like.

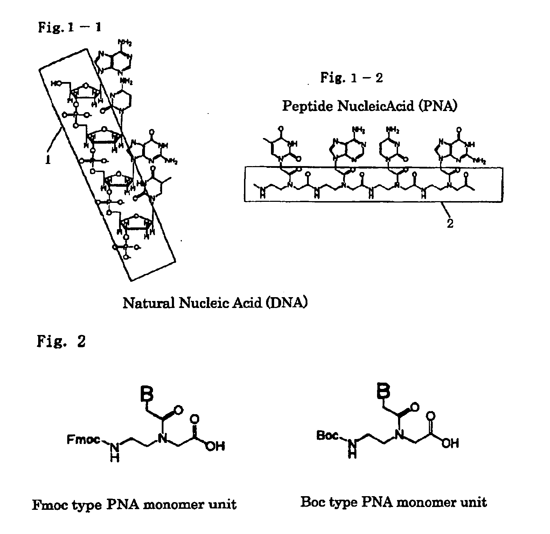

In particular, as shown in Fig. 1, PNA (Peptide Nucleic Acid) has the structure in which the sugar phosphoric acid skeleton in a natural nucleic acid such as DNA is converted into the N-(2-aminoethyl)glycine skeleton, is high in a double strand formation performance and a base sequence recognition performance compared with a natural nucleic acid, further is intact for an *in vivo* nuclease and protease, and therefore its application to a gene therapy as an antisence molecule is examined, attracting attention in recent years. The above characteristics of PNA is due to the fact that the sugar phosphoric acid skeleton in a natural nucleic acid has negative charge in neutral conditions whereby an electrostatic repulsion between complementary chains is produced, and in contrast in PNA having N-(2-aminoethyl)glycine skeleton without charge no electrostatic repulsion is produced between complementary chains.

Synthesis of PNA is carried out by sequentially combining an amino acid (especially glycine) derivative (monomer unit) which is introduced by any one of four kinds of bases (A, T(U), C and G) constituting DNA or RNA according to an aimed base sequence using a conventional solid peptide synthesis method. As for monomer units for the synthesis of PNA, there are two types, Fmoc type and Boc type, as shown in Fig. 2 (B represents base), though synthetic methods of monomer units are established and use of a Fmoc type which, enables to utilize a general DNA automatic synthesis machine to synthesize a PNA oligomer currently has become a major trend. However, because in case of synthesizing PNA by use of a Boc type monomer unit there is an advantage that a functional molecule unstable in basic conditions can be introduced in PNA, establishment of a PNA synthesis method using a Boc type monomer unit becomes an urgent matter.

As one of obstacles to hinder establishment of a PNA synthesis method using a Boc type monomer unit, it can be cited that a simple and cheap synthesis method for a monomer unit before introduction of a base, that is, a Boc type amino acid derivative shown in the formula I, is not to be established. Also, because an amino acid derivative of the formula I has use as a base substance for introducing other functional molecule in stead of the base, synthesis of an amino acid derivative introducing a functional molecule becomes easy if its simple and cheap synthesis method is established.

The synthetic method for an amino acid derivative of the formula I usually makes ethylenediamine a starting material and contains a step to introduce t-butoxycarbonyl group (Boc) to one nitrogen atom and a step to introduce - CHR¹-COOH to the other nitrogen.

As a method to obtain t-butoxycarbonylaminoethylamine by introduction of Boc to one nitrogen atom of ethylenediamine, for example, (1) methods are reported in which t-butoxycarboxylic acid anhydride is directly reacted to ethylenediamine in a reaction solvent such as chloroform, methanol or dioxane (J. Med. Chem., 38(22), 4433-8; 1995, Bull. Korean Chem. Soc., 15(12),1025-7; 1994, Eur. J. Med. Chem., 26(9), 915-20; 1991, Synth. Commun., 20(16), 2559-64; 1990, Aust. J. Chem., 39(3), 447-55; 1986), 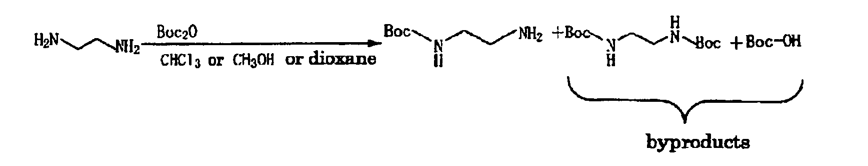 and (2) a method in which t-butoxycarboxylic acid anhydride is converted to an active ester followed by reaction with ethylenediamine (JP, A 11-012234,).

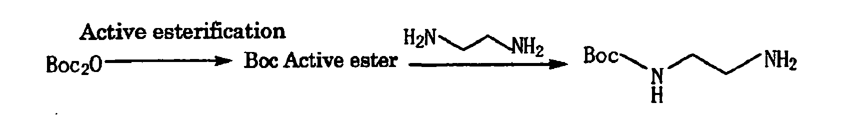

Also, as a method to obtain an amino acid derivative of the formula I by introduction of -CHR¹-COOH to t-butoxycarbonylaminoethylamine, a method is reported in which benzyl group is introduced to the unprotected nitrogen atom of t-butoxycarbonylaminoethylamine, (3) followed by reaction with benzyl bromoacetate and then by the catalytic reduction (J. Org. Chem., 62(2), 411-416; 1997).

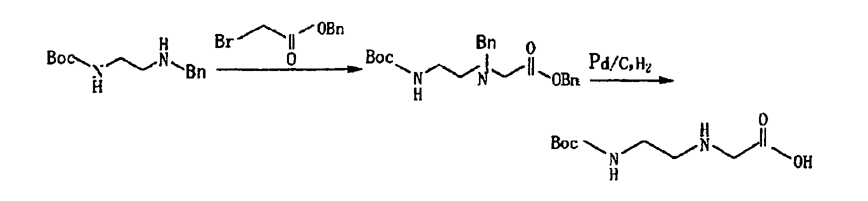

Further, as a method to obtain an amino acid derivative of the formula I by introduction of Boc to the other nitrogen atom of the ethylenediamine derivative in which -CHR¹-COOH is introduced to one nitrogen atom, (4) a method is reported in which t-butoxycarboxylic acid anhydride is reacted to N-(2-aminoethyl)glycine (Heimer, E. P.; Gallo-Torres, H. E.; Felix, A. M.; Ahmad, M.; Lambros, T. J.; Scheidl, F.; Meienhofer, J.Int. J. Pept. Protein Res. 23(2), 203-211, 1984).

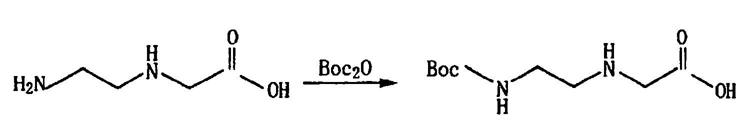

However, as a method to prepare t-butoxycarbonylamino-ethylamine, in the method 1 the aimed substance can be obtained in relatively good yield, though di(t-butoxycarbonylamino)ethylene and t-butoxycarboxylic acid are produced as byproducts, existing in a reaction solvent such as chloroform, methanol or dioxane. Owing to this, a partition extraction procedure or partition chromatography are necessary, making it difficult to prepare t-butoxycarbonylaminoethylamine efficiently in a large amount and cheaply.

Also, although the method 2 has an advantage that di(t-butoxycarbonylamino)ethylene is not produced as a byproduct, the total yield is as low as about 60% due to a multistep reaction, and because used reagents must be removed by partition chromatography, it is difficult to prepare t-butoxycarbonylaminoethylamine efficiently in a large amount and cheaply just like the method 1.

Therefore, both methods 1 and 2 are inappropriate as a method to industrially prepare t-butoxycarbonylaminoethylamine.

Also, as a method to obtain an amino acid derivative of the formula I from t-butoxycarbonylaminoethylamine, the method 3 is a multistep reaction, and a partition extraction procedure is necessary, being inappropriate for an industrial preparation.

Further, as a method to obtain an amino acid derivative of the formula I, the method 4 has an advantage that partition chromatography is unnecessary, though the yield is around 60%, being inappropriate for an industrial preparation. Thus, there is no efficient method to obtain a photo-functional PNA molecule.

### Summary of the Invention

The present invention has as its object a process to prepare an amino acid derivative of formula I and its synthetic intermediate, t-butoxycarbonylaminoethylamine, which is not tedious, gives good yield, and is readily applicable to mass production.

A first aspect of the present invention is a compound of formula (IV) 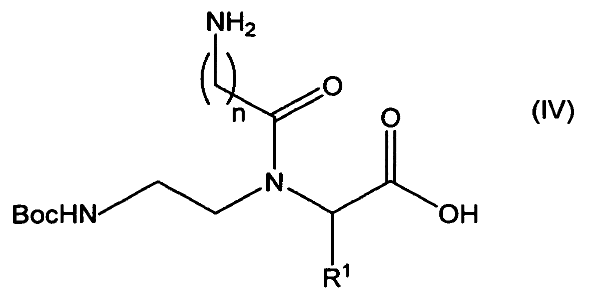 wherein R¹ is H or C₁₋₅ alkyl, and n is an integer of 1-11.

A second aspect of the present invention is a compound of formula (VIa) 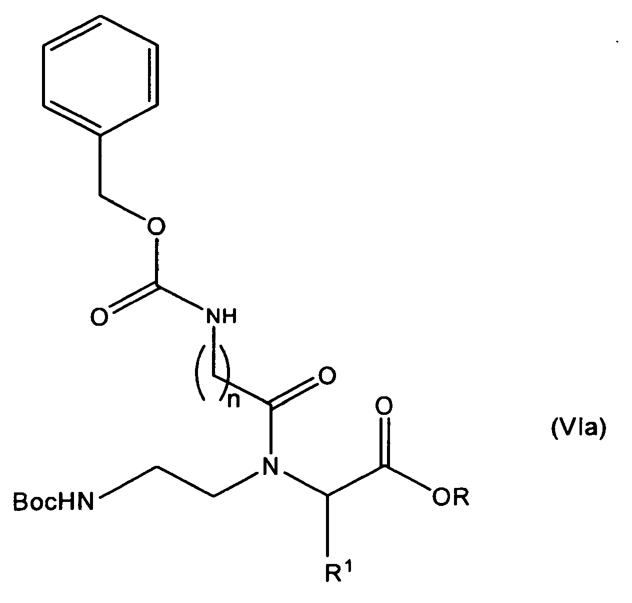 wherein R¹ and n are as defined above, and R is H or C₁₋₄ alkyl, e.g. ethyl. These compounds include those of formulae (V), i.e. wherein R is H, and (VI), i.e. wherein R is the group R², i.e. C₁₋₄ alkyl, as shown below.

A further aspect of the present invention is the use of a compound of formula (IV) or (VIa) as a base in the synthesis of a Bnc-type PNA monomer unit.

A compound of formula (IV) may be prepared by reduction of a compound of formula (VIa) wherein R is H. Preferably, the reaction is carried out in a methanol solution containing palladium on carbon as a catalyst.

A compound of formula (VIa) wherein R is H may be prepared by hydrolysis of the corresponding compound wherein R is alkyl. The hydrolysis preferably comprises reaction with aqueous alkali metal hydroxide. Preferably, in addition, alkali metal ion is removed by cation-exchange chromatography using pyridinium ion as a counter-ion. The alkali metal is preferably lithium, sodium or potassium.

A compound of formula (VIa) wherein R is alkyl may be prepared by the reaction of a compound of formula (VII) 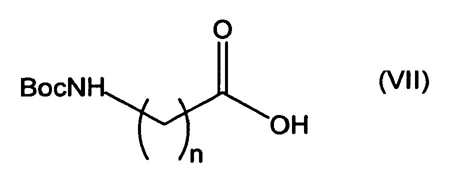 wherein n is as defined above with a compound of formula (II) 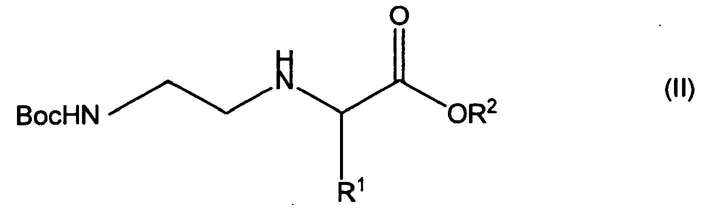 wherein R¹ is as defined above, and R² is C₁₋₄ alkyl.

A compound of formula (II) may be prepared by reaction of t-butoxycarbonylamino-ethylamine and a compound of formula (III) 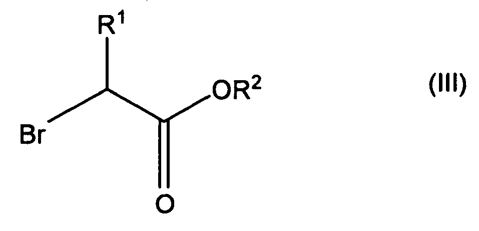 wherein R¹ and, R² are as defined above.

Preferably, R¹ is H, R² is ethyl and n is 1.

Compounds of formula (IV) are versatile intermediates. A target PNA monomer unit can be obtained in one step, by reaction of an active ester with the compound. Since such a photo-functional molecule can be converted into a PNA monomer unit using fewer synthetic steps than known methods, the invention provides an effective means of targeting relatively expensive photo-functional molecules.

A photo-functional molecule which is of the sulfonic acid chloride type and which provides large steric hindrance can be converted into a PNA monomer using compounds of formula (I). Various functional PNA monomers can be synthesised, by virtue of the present invention.

### Brief Description of the Drawings

Fig. 1-1 and Fig. 1-2 show the structures of a natural nucleic acid and a peptide nucleic acid. Fig. 2 shows a Fmoc-type monomer unit and a Boc-type monomer unit. Each shows a sugar phosphoric acid skeleton and a N-(2-aminoethyl)glycine skeleton, respectively.

### Embodiment of the Invention

An embodiment of the invention will now be described in more detail.

Amino acid derivatives shown by formula (I) are prepared by the steps shown below.

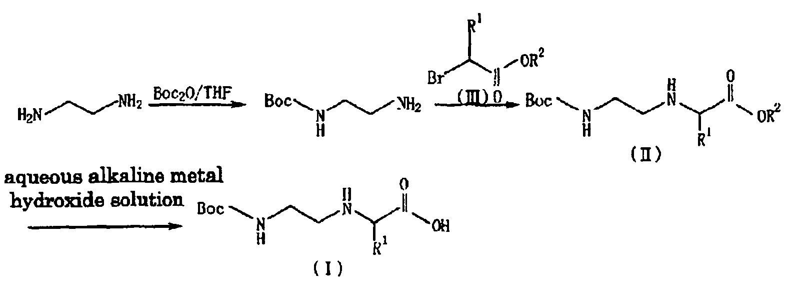

The first step is the step wherein t-butoxycarboxylic acid anhydride is directly reacted with ethylenediamine using tetrahydrofuran (THF) as solvent without using a reagent to prepare t-butoxycarbonylaminoethylamine.

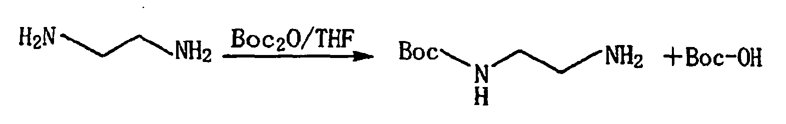

t-Butoxycarbonylaminoethylamine can be obtained in a high yield of 98% without producing di-(t-butoxycarbonylamino)ethylene as a byproduct. Since t-butoxycarboxylic acid produced as the byproduct is removed from the reaction system by forming itself into salts insoluble in THF with excessively existing ethylenediamine, purification by partition chromatography is unnecessary.

Therefore, it is possible to prepare simply t-butoxycarbonylaminoethylamine in a high yield, and further application to a large scale production is easy due to unnecessariness of purification by partition chromatography. Further, since excessively existing ethylenediamine and THF used as the solvent can be reused after distillation, it is possible to attempt a production cost reduced, making use of resource effectively. Further, in the above reaction the reaction condition is preferably under nitrogen atmosphere at room temperature.

The subsequent step is the step that the compounds shown by the general formula (II) are prepared by the reaction of t-butoxycarbonylaminoethylamine and the esters shown by the formula (III).

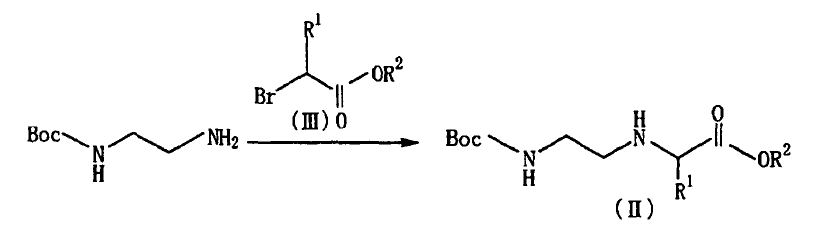

In the above reaction, though as solvents that can be used are chloroform, THF and the like, use of methylene chloride is preferable. Also, as a catalyst tertiary amine, especially triethylamine, diisopropylethylamine and the like can preferably be used. The reaction condition is preferably under a nitrogen atmosphere at room temperature.

Although it is determined that the amino acid derivatives shown by the formula (I), which are the final products, become any amino acid derivative in accordance with the type of R¹ in the esters shown by the formula (III), the yield of the above reaction is reduced by steric hindrance depending on the type of R¹. Therefore, R¹ is preferably a hydrogen atom or a straight chain or branched chain alkyl group with 1-5 carbon atoms, more preferably a hydrogen atom or a straight chain or branched chain alkyl group with 1-4 carbon atoms, furthermore preferably a hydrogen atom, a methyl or ethyl group. Further, R² is preferably a straight chain or branched chain alkyl group with 1-4 carbon atoms, more preferably a methyl, ethyl, n-propyl or isopropyl group, furthermore preferably an ethyl group.

The subsequent step is the step that the amino acid derivatives shown by the formula (I) are prepared by hydrolysis of the compounds shown by the formula (II).

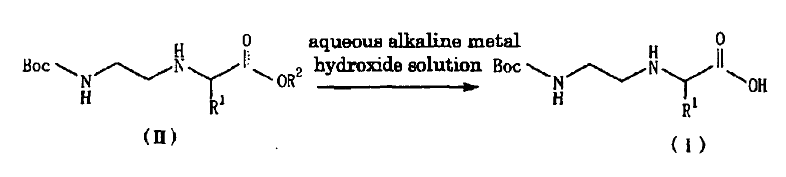

The hydrolysis is preferably carried out in an aqueous alkaline metal hydroxide solution. As an alkaline metal, lithium, sodium or potassium are preferable, and sodium is most preferable.

Although the amino acid derivatives shown by the formula (I) are obtained in a form of sodium salts by the above reaction, a sodium ion can easily be removed by cation-exchange chromatography. Further, since a t-butoxycarbonyl group is unstable toward cation-exchange chromatography, it is preferable to remove an alkaline metal ion by cation-exchange chromatography using a pyridinium ion instead of proton as a counter ion in view of preventing decrease of yield.

Since this step is a simple one step reaction called hydrolysis and does not require purification by column chromatography of which application to a mass production is difficult, the amino acid derivatives shown by the formula (I) can be prepared in a high yield, and a further application to an industrial production is easy.

The process of t-butoxycarbonylethyaminoethylamine and the amino acid derivatives of the invention does not use alkaline conditions. In the meantime, there are many functional molecules except bases constituting a nucleic acid, which are unstable toward an alkaline condition. Therefore, the process of the invention is preferably used in case of preparing amino acid derivatives in aiming their use as base substances for introducing functional molecules.

The amino acid derivatives shown by the formula (IV) of the invention are prepared by the steps shown below using the compounds shown in the previous general formula (II).

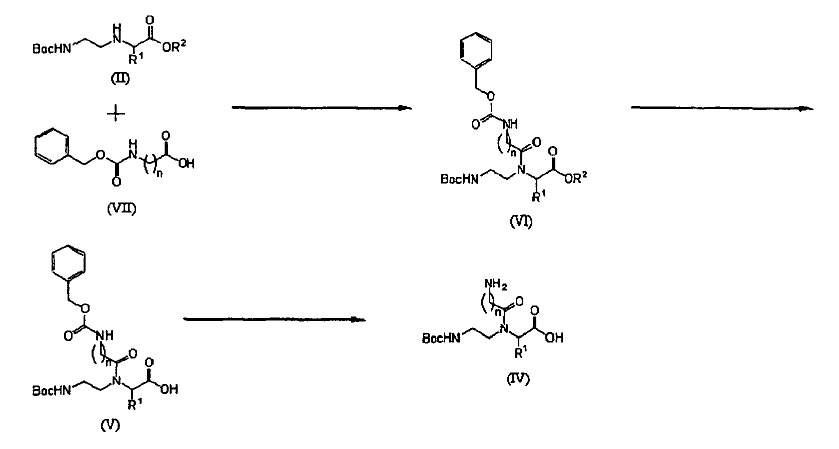

The first step is , as described below, the step to prepare the benzyloxycarbonyl- -amino acid^{BOC}PNA-OR² by the reaction of the compound ^{BOC}PNA-OR² shown by the general formula (II) with the benzyloxycarbonyl- - amino acid shown by the general formula (VII) using dimethylformamide (DMF) or the like as the solvent and triethylamine.

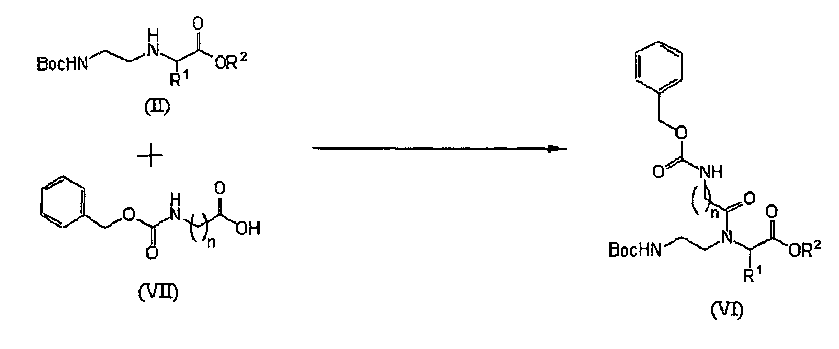

The solvent DMF, the compounds (II), (VII) and the EDCI derived product can be separable from the aimed substance (VI) by a partition procedure. Since theoretically only the aimed substance (VI) remains in an organic layer, purification by column is unnecessary, though just to be sure purification was done. The aimed substance was obtained quantitatively by this method.

The subsequent step is the step to prepare the benzyloxycarbonyl- -amino acid-^{BOC}PNA-OH shown by the general formula (V) by hydrolysis of the benzyloxycarbonyl- -amino acid^{BOC}PNA-OR² shown by the general formula (VI).

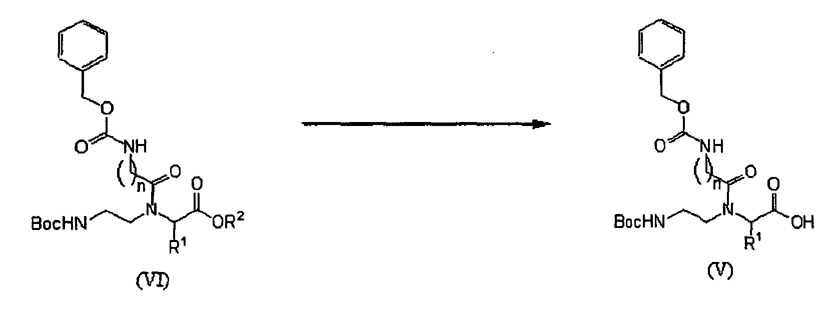

The hydrolysis is preferably carried out in an aqueous alkaline metal hydroxide solution. As an alkaline metal, lithium, sodium or potassium are preferable, and in particular sodium is preferable. Further, the condition of hydrolysis is under ice-cooling or at room temperature.

Subsequently, the step to obtain the compound, the final step, shown by the general formula (IV) is carried out.

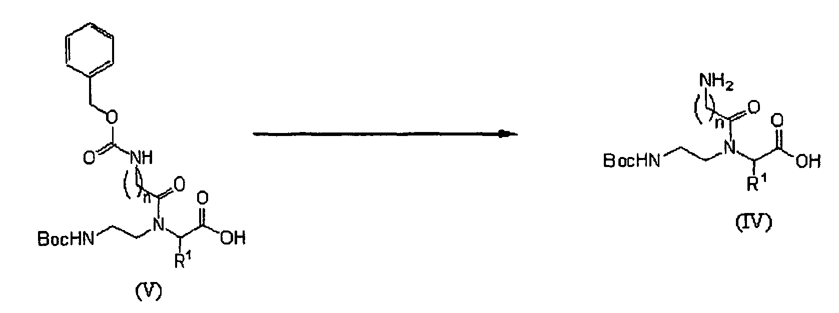

This step is preferably carried out in a methanol solution containing palladium carbon as a catalyst.

As a method to synthesize the compound (IV) from the compound (VI), the method via (V) is the only method. For example, as in the following figures, in case (VI) is first subjected to a catalytic reduction, the cyclic compound is formed via the intermediate.

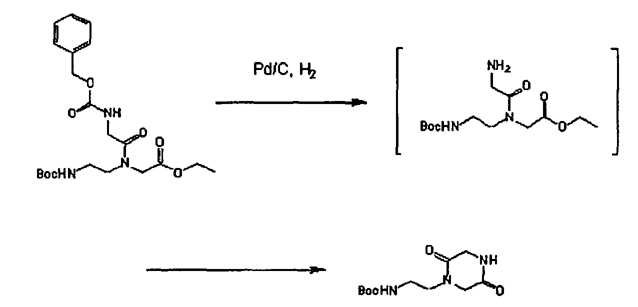

Although the amino acid derivative shown by the formula (IV) is obtained in the form of sodium salt by the above reaction, the sodium ion is easily removed by cation-exchange chromatography. Further, since t-butoxycarbonyl group is unstable toward cation-exchange chromatography, it is preferable that an alkaline metal ion is removed by cation-exchange chromatography using a pyridinium ion instead of proton as a counter ion in view of preventing decrease of yield.

Since this step is a simple one step reaction called hydrolysis and does not require column chromatography difficult for the application to a mass production, the amino acid derivatives shown by the formula (IV) can be prepared in a high yield, and further the application to a mass production is easy.

The process of the amino acid derivative of the invention does not use an alkaline condition. In the meantime, there are many functional molecules except bases constituting a nucleic acid, which are unstable toward alkaline conditions. Therefore, the process of the invention is preferably used in case of preparing amino acid derivatives in aiming their use as base substances for introducing functional molecules.

It is determined that the amino acid derivative shown by the formula (IV), which are the final product, becomes any amino acid derivative in accordance with a type of R¹ and value of n in the ester shown by the formula (VI).

The yield of the above reaction is reduced by steric hindrance depending on the type of R¹. Therefore, R¹ is preferably a hydrogen atom or a straight chain or branched chain alkyl group with 1-5 carbon atoms, more preferably a hydrogen atom or a straight chain or branched chain alkyl group with 1-4 carbon atoms, furthermore preferably a hydrogen atom, a methyl or ethyl group. Further, R² is preferably a straight chain or branched chain alkyl group with 1-4 carbon atoms, more preferably a methyl, ethyl, n-propyl or isopropyl group, and furthermore preferably an ethyl group.

Further, in order to synthesize the compound of the general formula (IV), which is different in n, the corresponding benzyloxycarbonyl- -amino acid can be used. Generally, since the benzyloxycarbonyl- -amino acids of n=1-11 are commercially available, it is easy to obtain them. Names thereof are as follows.

| | |
|---|---|
| n | Upper column:general name |
| | Lower column:rational formula |
| n = 1 | N-Benzyloxycarbonylglycine |
| | Z-NH-CH₂-COOH |
| n = 2 | N-Benzyloxycarbonyl-β-alanine |
| | Z-NH-(CHO₂)₂-COOH |
| n = 3 | N-Benzyloxycarbonyl-4-aminobutanoic Acid |
| | Z-NH-(CH₂)₃-COOH |
| n = 4 | N-Benzyloxycarbonyl-6-aminopentanoic Acid |
| | Z-NH-(CH₂)₄-COOH |
| n = 5 | N-Benzyloxycarbonyl-6-aminocaproic Acid |
| | Z-NH-(CH₂)₅-COOH |
| n = 6 | N-Benzyloxycarbonyl-7-aminoheptanoic Acid |
| | Z-NH-(CH₂)₆-COOH |
| n = 7 | N-Benzyloxycarbonyl-8-aminooctanoic Acid |
| | Z-NH-(CH₂)₇-COOH |
| n = 8 | N-Benzyloxycarbonyl-9-aminononanoic Acid |
| | Z-NH-(CH₂)₈-COOH |
| n = 9 | N-Benzyloxycarbonyl-10-aminodecanoic Acid |
| | Z-NH-(CH₂)₉-COOH |
| n = 10 | N-Benzyloxycarbonyl-11-aminoundecanoic Acid |
| | Z-NH-(CH₂)₁₀-COOH |
| n = 11 | N-Benzyloxycarbonyl-12-aminododecanoic Acid |
| | Z-NH-(CH₂)₁₁-COOH |

Among these, Z-glycine, n=1, can in particular preferance, be used.

Since generally PNA is expected to be a hybrid with DNA, derivatization sterically similar to DNA is desirable. In case the carboxylamino acid is used as a linker, Z-glycine is most preferable considering this point.

### Example

The invention will be illustrated in more detail by way of examples, but the invention is not limited to these examples.

### (Example 1) Preparation of t-butoxycarbonylaminoethylamine

To the THF solution (600ml) of ethylenediamine (90.2g, 1.50mol) was dropped the THF solution (400ml) of t-butoxycarboxylic acid anhydride (32.7g, 0.15mol) in 1 hour, and the mixture was stirred at room temperature for 15 hours. Subsequently, the supernatant is poured and evaporated under reduced pressure at 60°C. The residue was redissolved in THF and dried over MgSO₄, followed by filtration. The filtrate was concentrated to give t-butoxycarbonylaminoethylamine 23.7g as a colorless oil. The yield was 98%.

### (Example 2) Synthesis of ^{BOC}PNA-OH

To the THF solution (5ml) of ethyl N-(2-Bocaminoethyl)glycinate (^{BOC}PNA-OEt; 4.52g, 18.4mmol) obtained by reaction of t-butoxycarbonylaminoethylamine with ethyl bromoacetate was dropped aqueous 2N-NaOH solution (10ml, 20mmol) at 0°C, and then the reaction liquid was stirred at room temperature for 15 hours. This was directly subjected to cation-exchange chromatography (DOWEX 50W×8, pyridinium form) and eluted with water. The eluate was concentrated under reduced pressure and further dried in vacuum to give N-(2-Bocaminoethyl)glycine (^{BOC}PNA-OH) 3.85g as a white powder. The yield was 96%.

### (Example 3) Synthesis of Z-gly-^{BOC}PNA-OEt

To the dimethylformaamide solution (DMF; 25ml) of benzyloxycarbonylglycine (Z-glycine; 6.75g, 33 mmol) and ethyl N (2-aminoethyl)glycine (4.06g, 17mmol) was added triethylamine (TEA; 4.78ml, 35 mmol) and the mixture was stirred at 0°C. This was added with 1-ethyl-(3-(3-dimethylaminopropyl)carbodiimide (EDCI; 6.79g, 35mmol) and stirred at 0°C for 2 hours and further at room temperature for 15 hours. The reaction liquid was added with ethyl acetate (EtOAc; 300ml) and sequentially washed with aqueous 5% sodium bicarbonate solution (NaHCO3; 300ml×3), aqueous 5% citric acid solution (300ml×3), aqueous saturated sodium chloride solution (300ml×3). The EtOAc layer was dried over anhydrous magnesium sulfate (MgSO₄) and then filtered whereby the filtrate was concentrated. The residue was subjected, to silicagel column chromatography (3% MeOH/dichloromethane) to obtain quantitatively Z-gly-^{BOC}PNA-OEt as a colorless oil. ¹H NMR (CDCl₃) 7.4-7.2 (m, 5H), 5.77 (brt) and 5.68 (brt) (1H), 5.39 (brs) and 4.97 (brs) (1H), 5.27 (s) and 5.09 (s) (2H), 4.19 (m, 2H), 4.07 (s) and 3.91 (s) (2H), 4.01 (s, 2H), 3.51 (brs) and 3.40 (brs) (2H), 3.34 (brs) and 3.25 (brs) (2H), 1.40 (s, 9H), 1.26 (t, *J*=7.2 Hz, 3H); ¹³C NMR (CDCl₃) 169.71 and 169.31 (3), 169.20 and 168.79 (d), 156.11 and 155.85 (d), 136.39 and 136.32 (d), 128.44, 128.27, 127.98, 127.90, 79.80 and 79.37 (d), 66.86 and 66.77 (d), 62.05 and 61.58 (d), 49.43 and 48.73 (d), 48.52 and 48.05 (d), 42.49 and 42.34 (d), 38.48, 28.27, 14.03; FABMS *m*/*z* 438 [(M+H)⁺].

### (Example 4) Synthesis of Z-gly-^{BOC}PNA-OH

To the THF solution (20ml) of Z-gly-^{BOC}PNA-OEt (4.0g, 9.2mmol) was dropped aqueous 1N-NaOH solution (20ml, 20mmol) at 0°C, and the reaction liquid was stirred at 0°C for 1 hour. After the reaction was completed, the reaction liquid was directly allowed to cation-exchange chromatography (DOWEX 50W×8, pyridinium form) and eluted with MeOH. The eluate was concentrated under reduced pressure and further dried in vacuum to obtain Z-gly-^{BOC}PNA-OH (3.09g, 82%) as a colorless oil. ¹H NMR (DMSO-*d6*) 7.4-7.2 (m, 5H), 6.84 (brt) and 6.73 (brt) (1H), 5.03 (s) (2H), 4.11 (brs) and 3.94 (brs) (2H), 3.92 (brs) and 3.77 (brs) (2H), 3.33 (brs) and 3.29 (brs) (2H), 3.09 (brs) and 3.02 (brs) (2H),1.37 (s, 9H); ¹³C NMR (DMSO-*d6*) 171.07 and 170.02 (d), 169.39 and 169.07 (d), 156.42 (brd), 155.70 and 155.61 (d), 137.14, 128.35, 127.77, 127.67, 78.04 and 77.76 (d), 65.38, 48.99, 47.43 and 46.70 (d), 41.92 and 41.52 (d), 38.13 and 37.81 (d), 28.23; FABMS *m*/*z* 410 [(M+H)⁺]; HRMS (FAB⁺) calcd for C₁₉H₂₈O₇N₃ [(M+H)⁺] 410, 1849, observed 410, 1926.

### (Example 5) Synthesis of Gly-^{BOC}PNA-OH

To the MeOH solution (20ml) of Z-gly-^{BOC}PNA-OH (4.09g, 10mmol) was added palladium carbon (5% Pd/C; 100mg), and the catalytic hydrogen reduction was carried out at room temperature. After the reaction was completed, the mixture was filtered through celite. The residue was subjected to silicagel column chromatography (5% MeOH/dichloromethane) to obtain Gly-^{BOC}PNA-OH (2.08g, 75%) as white powder. ¹H NMR (DMSO-*d6*) 3.72 (brs) and 3.69 (brs) (2H), 3.58 (brs) and 3.54 (brs) (2H), 3.3-3.2 (m, 2H), 3.06 (brs) and 2.94 (brs) (2H); FABMS *m*/*z* 276 [(M+H)⁺].

### (Example 5) Synthesis of Dabcyl-Gly-^{BOC}PNA-OH

To the dimethyformamide solution (10ml) of Gly-^{BOC}PNA-OH (100mg, 0.39mmol) was added dabcyl *N*-hydroxysuccinimide ester (145mg, 0.40mmol) and triethylamine (60µl, 0.45mmol) sequentially, and the mixture was stirred at room temperature for 15 hours. After the reaction was completed, the mixture was concentrated under reduced pressure, and the residue was subjected to silicagel column chromatography (0.4% MeOH/dichloromethane) to obtain Dabcyl-Gly-^{BOC}PNA-OH (184mg, 90%) as red brown powder. ¹H NMR (DMSO-*d6*) 8.18 (d, J=7Hz, 2H), 7.91 (d, J=7Hz, 2H), 7.88 (d, J=7Hz, 2H), 6.77 (d, J=7Hz, 2H), 5.76 (s) and 5.30 (s) (2H), 4.22 (brs) and 4.05 (brs) (2H), 3.73 (brs) and 3.49 (brs) (2H), 3.47 (brs) and 3.29 (brs) (2H),1.26 (s, 9H); FABMS *m*/*z* 527 [(M+H)⁺].

### Industrial Applicability

The process of the invention is able to synthesize t-butoxycarbonylaminoethylamine and Boc type amino acid derivatives whereby it requires no tedious procedure and is also good in yield, and the application to a mass production is easy. Therefore, it can be used for an industrial synthesis of t-butoxycarbonylaminoethylamine and Boc type amino acid derivatives, and utilized for the establishment of a PNA synthesis method using Boc type monomer units, and in the industries of an industrial synthesis of Boc type amino acid derivatives, an industrial synthesis of PNA monomer units using these and so on.

## Claims

1. A compound of formula (IV) 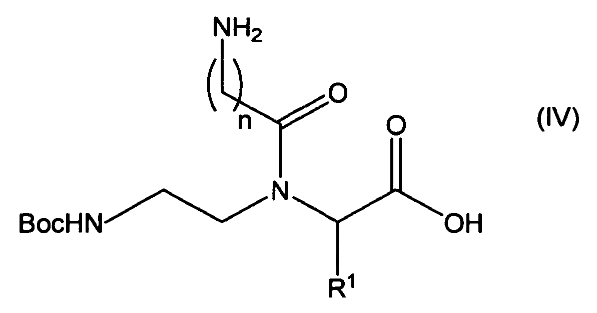 wherein R¹ is H or C₁₋₅ alkyl, and n is an integer of 1-11.

2. A compound of claim 1, wherein R¹ is H and n is 1.

3. A compound of formula (VIa) 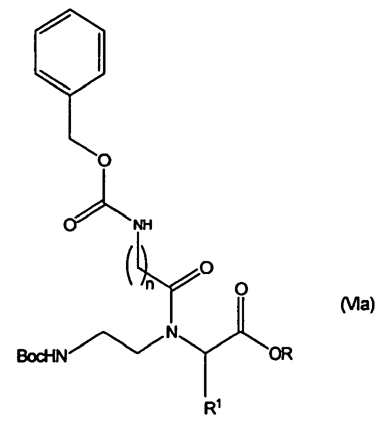 wherein R¹ and n are as defined in claim 1 or claim 2, and R is H or C₁₋₄ alkyl, e.g. ethyl.

4. Use of a compound of any preceding claim, as a base in the synthesis of a Boc-type PNA monomer unit.

5. A process for preparing a compound of claim 1 or claim 2, which comprises the reduction of a compound of claim 3 wherein R is H.

6. A process according to claim 5, wherein the reaction is carried out in a methanol solution containing palladium on carbon as a catalyst.

7. A process according to claim 5 or claim 6, which comprises the prior step of preparing the compound of claim 3 wherein R is H by hydrolysis of a compound of claim 3 wherein R is alkyl.

8. A process according to claim 7, wherein the hydrolysis comprises reaction with aqueous alkali metal hydroxide.

9. A process according to claim 8, which additionally comprises the removal of alkali metal ion by cation-exchange chromatography using pyridinium ion as a counter-ion.

10. A process according to claim 8 or claim 9, wherein the alkali metal is lithium, sodium or potassium.

11. A process according to any of claims 7 to 10, which comprises the prior step of preparing the compound of claim 3 wherein R is alkyl, by the reaction of a compound of formula (VII) 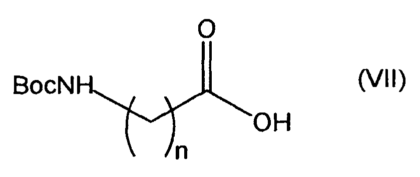 wherein n is as defined in claim 1 or claim 2 with a compound of formula (II) 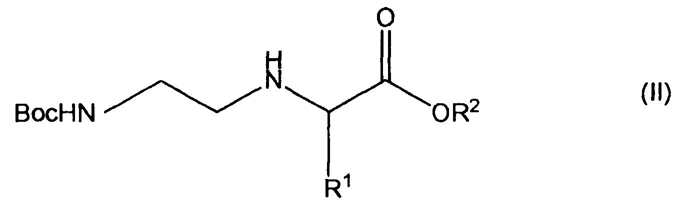 wherein R¹ is as defined in claim 1 or claim 2, and R² is C₁₋₄ alkyl.

12. A process according to claim 10, which comprises the prior step of preparing the compound of formula (II) by reaction of t-butoxycarbonylaminoethylamine and a compound of formula (III) 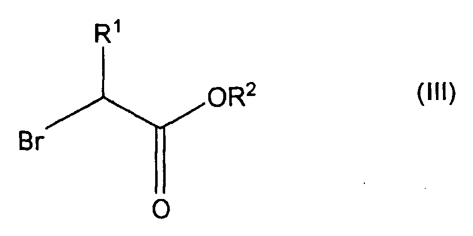 wherein R¹ is as defined in claim 1 or claim 2, and R² is as defined in claim 11.

13. A process for preparing a compound of claim 3, wherein R is H, which comprises hydrolysis of a compound of claim 3 wherein R is alkyl.

14. A process for preparing a compound of claim 3 wherein R is alkyl, which comprises reaction of a compound of formula (VII) as defined in claim 11 with a compound of formula (II) as defined in claim 11.

## Patentansprüche

1. Verbindung der Formel (IV) 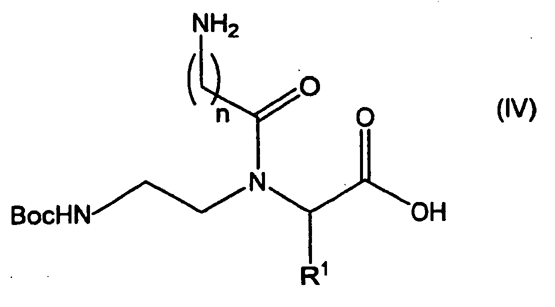 wobei R¹ H oder C₁₋₅ Alkyl ist, und n eine ganze Zahl von 1 bis 11 ist.

2. Verbindung nach Anspruch 1, wobei R¹ H ist und n 1 ist.

3. Verbindung der Formel (VIa) 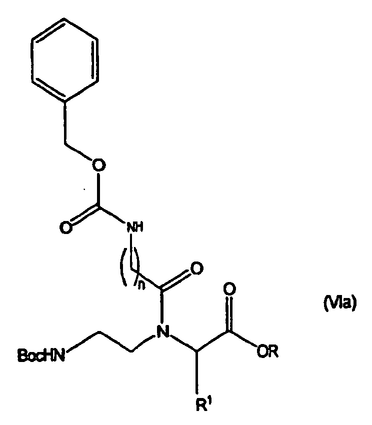 wobei R¹ und n wie in Anspruch 1 oder Anspruch 2 definiert sind, und R H oder C₁₋₄ Alkyl, beispielsweise Ethyl, ist.

4. Verwendung einer Verbindung nach einem vorhergehenden Anspruch als eine Base in der Synthese einer PNA-Monomereinheit vom Boc-Typ.

5. Verfahren zur Herstellung einer Verbindung nach Anspruch 1 oder Anspruch 2, welches die Reduktion einer Verbindung nach Anspruch 3 umfasst, wobei R H ist.

6. Verfahren nach Anspruch 5, wobei die Reaktion in einer Methanol-Lösung durchgeführt wird, welche Palladium auf Kohlenstoff als Katalysator enthält.

7. Verfahren nach Anspruch 5 oder Anspruch 6, welches den vorherigen Schritt des Herstellens der Verbindung nach Anspruch 3, wobei R H ist, durch Hydrolyse einer Verbindung nach Anspruch 3, wobei R Alkyl ist, umfasst.

8. Verfahren nach Anspruch 7, wobei die Hydrolyse die Reaktion mit wässrigem Alkalimetallhydroxid umfasst.

9. Verfahren nach Anspruch 8, welches zusätzlich das Entfernen von Alkalimetallionen durch Kationenaustauschchromatographie unter Verwendung eines Pyridiniumions als Gegenion umfasst.

10. Verfahren nach Anspruch 8 oder Anspruch 9, wobei das Alkalimetall Lithium, Natrium oder Kalium ist.

11. Verfahren nach einem der Ansprüche 7 bis 10, welches den vorherigen Schritt des Herstellens der Verbindung nach Anspruch 3, wobei R Alkyl ist, durch die Reaktion einer Verbindung der Formel (VII) 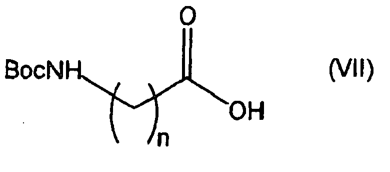 umfasst, wobei n wie in Anspruch 1 oder Anspruch 2 definiert ist, mit einer Verbindung der Formel (II) 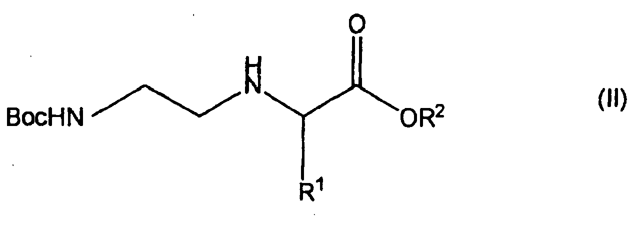 wobei R¹ wie in Anspruch 1 oder Anspruch 2 definiert ist, und R² C₁₋₄ Alkyl ist.

12. Verfahren nach Anspruch 10, welches den vorherigen Schritt des Herstellens einer Verbindung der Formel (II) durch Reaktion von t-Butoxycarbonylaminoethylamin und einer Verbindung der Formel (III) 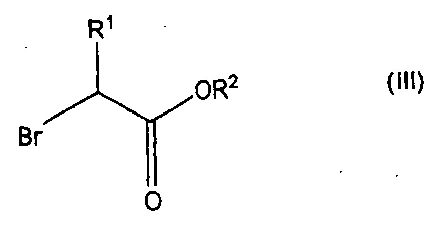 umfasst, wobei R¹ wie in Anspruch 1 oder Anspruch 2 definiert ist, und R² wie in Anspruch 11 definiert ist.

13. Verfahren zur Herstellung einer Verbindung nach Anspruch 3, wobei R H ist, welches die Hydrolyse einer Verbindung nach Anspruch 3, wobei R Alkyl ist, umfasst.

14. Verfahren zur Herstellung einer Verbindung nach Anspruch 3, wobei R Alkyl ist, welches die Reaktion einer wie in Anspruch 11 definierten Verbindung der Formel (VII) mit einer wie in Anspruch 11 defnierten Verbindung der Formel (II) umfasst.

## Revendications

1. Composé de la formule (IV) 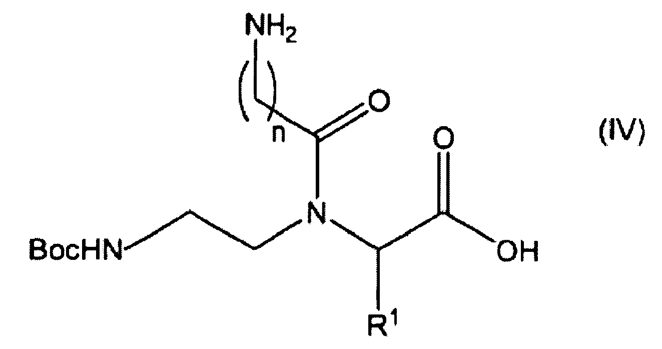 dans laquelle R¹ représente H ou un alkyle en C₁₋₅, et n est un entier de 1 à 11.

2. Composé selon la revendication 1, dans lequel R¹ représente H et n vaut 1.

3. Composé de la formule (VIa) 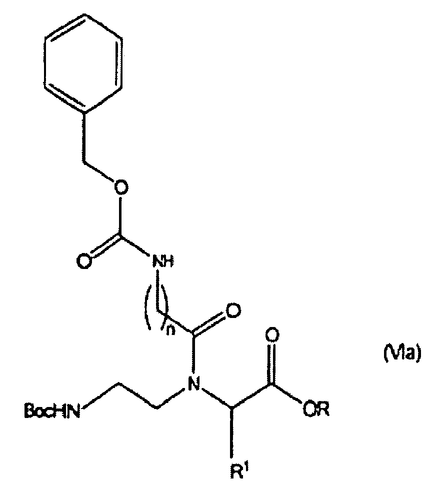 dans laquelle R¹ et n sont tels que définis à la revendication 1 ou à la revendication 2, et R représente H ou un alkyle en C₁₋₄, par exemple l'éthyle.

4. Utilisation d'un composé selon l'une quelconque des revendications précédentes, comme base dans la synthèse d'une unité monomère de PNA de type Boc.

5. Procédé de préparation d'un composé selon la revendication 1 ou la revendication 2, qui comprend la réduction d'un composé de la revendication 3 dans lequel R représente H.

6. Procédé selon la revendication 5, dans lequel la réaction est mise en oeuvre dans une solution de méthanol contenant du palladium sur carbone comme catalyseur.

7. Procédé selon la revendication 5 ou la revendication 6, qui comprend l'étape antérieure consistant à préparer le composé de la revendication 3 dans lequel R représente H, par hydrolyse d'un composé de la revendication 3 dans lequel R est un alkyle.

8. Procédé selon la revendication 7, dans lequel l'hydrolyse comprend une réaction avec un hydroxyde de métal alcalin aqueux.

9. Procédé selon la revendication 8, qui comprend de plus l'enlèvement de l'ion de métal alcalin par chromatographie par échange d'ions utilisant un ion pyridinium comme contre-ion.

10. Procédé selon la revendication 8 ou la revendication 9, dans lequel le métal alcalin est le lithium, le sodium ou le potassium.

11. Procédé selon l'une quelconque des revendications 7 à 10, qui comprend l'étape antérieure consistant à préparer le composé de la revendication 3 dans lequel R est un alkyle, par réaction d'un composé de la formule (VII) 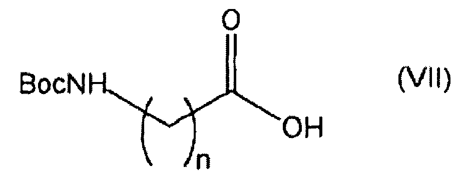 dans laquelle n est tel que défini à la revendication 1 ou à la revendication 2, avec un composé de la formule (II) 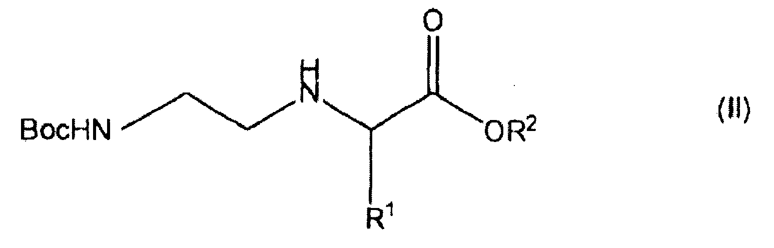 dans laquelle R¹ est tel que défini à la revendication 1 ou à la revendication 2, et R² est un alkyle en C₁₋₄.

12. Procédé selon la revendication 10, qui comprend l'étape antérieure consistant à préparer le composé de la formule (II) par réaction de t-butoxycarbonylaminoéthylamine et d'un composé de la formule (III) 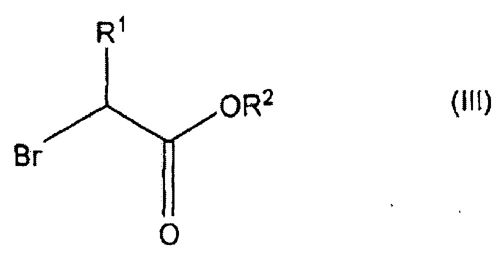 dans laquelle R¹ est tel que défini à la revendication 1 ou à la revendication 2, et R² est tel que défini à la revendication 11.

13. Procédé de préparation d'un composé selon la revendication 3, dans lequel R représente H, qui comprend l'hydrolyse d'un composé de la revendication 3 dans lequel R est un alkyle.

14. Procédé de préparation d'un composé selon la revendication 3, dans lequel R est un alkyle, qui comprend une réaction d'un composé de la formule (VII) tel que défini à la revendication 11 avec un composé de la formule (II) tel que défini à la revendication 11.
